# EUROPEAN PATENT SPECIFICATION

(11) **EP 0 740 932 B1**
(45) Date of publication and mention of the grant of the patent: **04.09.2002**
(21) Application number: 96201121.9
(22) Date of filing: 25.04.1996
(51) Int. Cl.: A61K 7/16, A61K 7/22

(54) **Clear gel-type dentifrices**
Klare Gelzahnpasta
Dentifrice sous forme de gel optiquement clair

(30) Priority: 03.05.1995 EP 95303028
(43) Date of publication of application: 06.11.1996
(60) Divisional application: 01205175.1
(73) Proprietor: UNILEVER N.V., 3000 DK Rotterdam (NL); UNILEVER PLC, London EC4P 4BQ (GB)
(72) Inventor: Riley, Paul Ian, Wirral, Merseyside L63 9AA (GB)
(74) Representative: van Gent, Jan Paulus

(56) References cited:
- EP-A- 0 162 574
- EP-A- 0 549 287
- GB-A- 2 052 978
- US-A- 4 992 259

## Description

The present invention relates to visually clear, gel-type dentifrices. More particularly, it relates to visually clear, gel-type dentifrices which contain a zinc salt.

Visually clear, gel-type dentifrices are known in the art, and have been used commercially. Such dentifrices usually comprise particular amorphous'synthetic silica xerogels in a liquid phase, the refractive indices of the silica xerogels and the liquid phase having about the same value. Such dentifrices are described e.g. in US-A-3,538,230 (Pader et al), US-A-3,689,637 (Pader), US-A-3,842,167 (Block et al), US-A-3,864,470 (Watson).

Dentifrices comprising a zinc salt are equally well-known, and have been described e.g. in US-A-4,022,880 (Vinson et al) and GB-A-1,373,001 (Pader et al).

GB-A-2 052 978 discloses oral compositions, particularly toothpastes comprising silica xerogel (Syloid 63), a zinc salt which is more soluble than zinc citrate, such as zinc chloride or zinc sulfate, and glycine.

EP-A-0 162 574 discloses dentifrices comprising zinc chloride, sodium fluoride, silica abrasives and sodium gluconate.

US-A-4 992 259 discloses stable and clear gel dentifrices comprising zinc chloride and sodium gluconate as zinc complexing agent.

However, the inclusion of a zinc salt in a visually clear, gel-type dentifrice poses significant problems as we have established in our attempts to make such dentifrices. The inclusion of a zinc salt such as zinc citrate in a visually clear, gel-type dentifrice resulted in a product which was not crystal clear; optical microscopy showed the presence of unsolubilised crystals of zinc citrate throughout the dentifrice. Incorporation of predissolved zinc citrate, nor the formation of zinc citrate in situ gave better dentifrices than with the direct incorporation of zinc citrate in the dentifrices. Attempts to include zinc salts which are more water-soluble than the sparingly soluble zinc citrate also failed to produce clear gel-type dentifrices.

We have now surprisingly found that the inclusion of a water-soluble zinc salt, and an amino acid which can bind zinc, in a gel-type dentifrice which contains as abrasive silica a silica with a low refractive index, results in a visually clear, gel-type dentifrice with excellent clarity.

Consequently, the invention relates to a visually clear, gel-type dentifrice which contains a zinc salt which is more water-soluble than zinc fluoride, an amino acid which can bind zinc, and a low refractive index type silica as abrasive agent in a liquid phase.
The invention will be discussed hereunder in more detail. The zinc salt should be more water-soluble than zinc fluoride (the solubility of which in 100 grammes water at 25°C is 1.62 grammes), i.e. should have a solubility of more than 1.02 grammes (expressed as zinc) per 100 mls of water at 25°C. Typical examples of suitable zinc salts are zinc acetate, zinc chloride, zinc lactate, zinc sulphate, zinc nitrate, zinc iodide, etc. and mixtures thereof. The preferred zinc salt is zinc sulphate (either anhydrous or as its heptahydrate). The amount of the zinc salt in the compositions of the present invention generally ranges from 0.1-10% by weight, calculated as zinc, usually from 0.2-5% by weight and preferably 0.5-2.5% by weight.

The amino acid used in the present invention should be capable of binding zinc, and should prevent the precipitation of zinc hydroxide at neutral pH, moving the boundary of zinc hydroxide precipitation to higher values. The theory and examples thereof are set out e.g. in the "Atlas of Metal-Ligand Equilibria in Aqueous Solutions" by J. Kragter, Ellis Horwood Ltd (1978), esp. pages 742 sqq..

The zinc binding of the amino acid should not be so strong as to inhibit the bio-activity of the zinc (see Cummins et al., J. Dent. Res., Nov. Sp. Issue, 1702 (1989) and Watson et al., Caries Res. 25, 431 (1991). Typical examples of suitable amino acids are N-dihydroxyethylglycine, glycine, glycylglycine, and aspartic acid. Glycine is the preferred amino acid, as it does not compromise the bioactivity of the zinc.

The amino acid should be used in excess of the amount, stochiometrically required to bind zinc in order to buffer the pH. The amount generally ranges from 0.01-5%, preferably 0.1-3% by weight of the composition.
It is observed that the combination of zinc sulphate and glycine has already been proposed for inclusion in oral care products such as mouth washes and toothpastes, e.g. in GB-A-2,052,978 (Unilever), but not with low refractive index (R.I.) silicas.

The low refractive index silicas, used as abrasives in the present invention are silicas with an apparent refractive index in the range of 1.41 - 1.47, preferably 1.435 - 1.445, preferably having a weight mean particle size of between 5 and 15 µm, a BET (nitrogen) surface area of between 10 and 100 m²/g and an oil absorption of about 70 - 150 cm³/100 g.

Typical examples of suitable low refractive index abrasive silicas having an R.I. of between 1.435 and 1.445 are Tixosil 63 and 73 ex Rhone Poulenc; Sident 10 ex Degussa; Zeodent 113 ex Zeofinn; Sorbosil AC 77 ex Crosfield Chemicals having an R.I. of approximately 1.440. Further examples can be found in EP-A-0,549,287 (Colgate) and in WO 94/10087 (Crosfield). The amount of these silicas in the composition generally ranges from 5-60% by weight, usually 5-20% by weight.

The liquid phase of the composition of the present invention should be formulated such, that its refractive index closely matches the refractive index of the silicas as discussed above. The liquid phase usually comprises water and a humectant, the latter usually being sorbitol or glycerol. Water has an RI of 1.333, sorbitol (70% aqueous solution) has an RI of 1.457 and glycerine an RI of 1.473. Other humectants such as propylene glycol, polypropylene glycol and polyethylene glycol may also be present. The refractive index of the liquid phase can be calculated from the respective refractive indices of the constituting ingredients.

The compositions of the invention may contain further, optional ingredients, as long as the clarity of the dentifrices is thereby not impaired and the RI of the liquid phase is about the RI of the low refractive index abrasive silica.

Thus the dentifrice may contain optional further ingredients such as pharmaceutically acceptable carriers like starch, sucrose, water or water/alcohol systems etc.. Small amounts of surfactants may also be included, such as anionic, nonionic and amphoteric surfactants. They may comprise thickening silicas and minor amounts of other particulate abrasive materials such as calcium carbonates, dicalciumphosphates, calcium pyrophosphates, hydroxyapatites, trimetaphosphates, insoluble hexametaphosphates and so on, but preferably the use of additional, insoluble abrasive materials should be avoided as they may compromise the clarity of the gel-type dentifrice. Such additional, insoluble abrasives may, however, be used in a coloured phase or stripes to provide a clear gel-type dentifrice with coloured stripes or a separate coloured phase.

Furthermore, the dentifrice formulations may comprise humectants such as glycerol, sorbitol, polyethyleneglycol, propyleneglycol, xylitol, lactitol and so on.
Binders and thickeners such as sodium carboxymethylcellulose, xanthan gum, gum arabic etc., may also be included, as well as synthetic polymers such as polyacrylates and carboxyvinyl polymers such as Carbopol®.

Flavours such as peppermint and spearmint oils may also be included, as well as preservatives, colouring agents, pH adjusting agents, sweetening agents and so on.

Anti-bacterial agents may also be included such as Triclosan, chlorhexidine, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-4(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate).

Furthermore anti-inflammatory agents such as ibuprofen, flurbiprofen, aspirin, indomethacin etc. may also be included.

Anti-caries agents such as sodium- and stannous fluoride, aminefluorides, monosodiumfluorophosphate, casein, plaque buffers such as urea, pyruvates, arginine, small peptides, calcium lactate, calcium glycerophosphate, strontium polyacrylates may also be included. A preferred anticaries agent is sodium monofluorophosphate, as fluorides may form crystals of zinc fluoride in the compositions of the invention under certain circumstances. Other optional ingredients include vitamins such as Vitamin C, and plant extracts. Desensitising agents such as potassium tartrate, potassium citrate, potassium chloride, potassium bicarbonate, potassium oxalate, potassium nitrate as well as strontium salts may also be included. When potassium salts are included, care should be taken in chosing a compatible surfactant, if required, sodium laurylsulphate e.g. not being suitable because that will cause precipitation of potassium laurylsulphate which compromises clarity. However, such incompatible surfactant can then e.g. be included in the coloured stripes or phase, mentioned before.

Buffers and salts to buffer the pH and ionic strength of the compositions may also be included. Liposomes and other encapsulates may also be used to improve delivery or stability of active ingredients.

Furthermore, the oral compositions may comprise anti-calculus agents such a alkalimetal pyrophosphates, hypophosphite-containing polymers, organic phosphonates, phosphocitrates etc.

In addition, the compositions may comprise functional biomolecules such as bacteriocins, antibodies, enzymes and so on.

Other optional ingredients that may be included are e.g. bleaching agents such as peroxy compounds e.g. potassium peroxydiphosphate, effervescing systems such as sodium bicarbonate/citric acid systems, colour change systems, and so on.

The dentifrices of the invention may also contain coloured particles suspended therein, e.g. coloured silica agglomerates or other coloured particles to impart a "speckled" appearance to the dentifrices. The dentifrices may also contain stripes of a coloured paste, to provide for a visually clear gel-type dentifrice with coloured stripes or a separate coloured phase.

The invention will further be illustrated by way of Examples.

### EXAMPLE 1

The following formulations were prepared:

| | A | B | C |
|---|---|---|---|
| Sorbosil AC 77 (abrasive low RI silica) | 10 | 10 | 10 |
| Sident 22 S(thickening silica) | 8 | 8 | 8 |
| Sorbitol (70%) | 58.59 | 58.95 | 70 |
| Polyethylene glycol MW 1500 | 5 | 5 | 5 |
| Sodium monofluorophosphate | 0.8 | - | - |
| Sodium fluoride | - | 0.33 | - |
| Sodium dodecylbenzenesulphonate | - | - | 0.5 |
| Sodium laurylsulphate | 1.7 | 1.7 | 1.7 |
| Sodium carboxymethylcellulose | 0.5 | 0.5 | 0.5 |
| Saccharin | 0.2 | 0.2 | 0.2 |
| Colouring agent | 0.012 | 0.012 | 0.3659 |
| Flavour | 1.1 | 1.1 | 1.1 |
| Zinc citrate (trihydrate) | - | - | 0.5 |
| Zinc sulphate heptahydrate | 0.686 | 0.686 | - |
| Glycine | 0.3584 | 0.3584 | - |
| Water | q.s. | q.s. | q.s. |

Variants of these formulations were also made, i.e: Formulation A1, containing 0.5% zinc citrate instead of zinc sulphate plus glycine, and A2 similar to A plus 0.3% Triclosan.

Formulation B1, containing 0.22% sodium fluoride instead of 0.33%, and B2, not containing any fluoride. Formulation C1, containing 0.686% zinc sulphate heptahydrate and 0.3584% glycine instead of 0.5% zinc citrate, and C2, not containg any zinc salt.
The clarity of these formulations were assessed as follows:

The formulation was transferred to a 1 cm path clear cell and placed on a light table above a numeric scale -12 to +13 where the size of the numbers smoothly decrease from 10 mm high (-12) to 0.25 mm low (+13). The clarity of the formulation was given the value to the smallest number that could be read.

The following results were obtained:

| | Clarity |
|---|---|
| C₂ | + 13 |
| C₁ | - 12 |
| C₁ | - 12 |
| A₁ | - 12 |
| A | + 13 |
| A₂ | + 13 |
| B | + 3 |
| B₁ | + 13 |
| B₂ | + 13 |

**EXAMPLE 2**

| **INGREDIENT** | **LEVEL (%)** | | | |
|---|---|---|---|---|
| | **D** | **E** | **F** | **G** |
| Sorbosil AC 77 (abrasive silica) | 10.00 | - | 10.00 | - |
| Tixosil 63 (abrasive silica) | - | 10.00 | - | 10.00 |
| Sorbitol (70%) | 58.00 | 58.00 | 59.00 | 57.00 |
| Sodium carboxymethylcellulose | 0.50 | 0.50 | 0.60 | 0.70 |
| Polyethylene glycol MW 1500 | 5.00 | 5.00 | 5.00 | 5.00 |
| Sodium fluoride | 0.33 | 0.33 | 0.33 | 0.33 |
| Sident 22S (thickening silica) | 8.00 | 8.00 | 8.00 | 8.00 |
| Zinc sulphate | 0.686 | 0.686 | 0.686 | 0.686 |
| Glycine | 0.3584 | 0.3584 | 0.3584 | 0.3584 |
| Sodium dodecylbenzene sulphate | 0.50 | 0.50 | 0.50 | 0.50 |
| Sodium lauryl sulphate | 1.70 | 1.70 | 1.70 | 1.70 |
| Saccharin | 0.20 | 0.20 | 0.20 | 0.20 |
| Yellow colouring agent | 0.014 | 0.014 | 0.014 | 0.014 |
| Blue colouring agent | 0.0017 | 0.0017 | 0.0017 | 0.0017 |
| Flavour | 1.20 | 1.20 | 1.20 | 1.20 |
| Water | 13.51 | 13.51 | 12.41 | 14.31 |

| **Initial Specifications** | | | | |
|---|---|---|---|---|
| Density (g/ml) | 1.34 | 1.34 | 1.33 | 1.35 |
| pH | 5.98 | 6.02 | 6.05 | 6.05 |
| Clarity | 20 | 20 | 20 | 20 |
| Cohesion | 140 | 110 | 180 | 180 |

Formulations were packed in laminate tubes and stored at 20°C, 28°C, 37°C, and 50°C with measurements of the clarity at 1, 2, 3 and 6 months. The clarity was measured as follows: The formulation was transferred to a clear cell with a path length of 0.5 cm and placed on a light table above a line scale. The line scale consisted of a series of broad lines becoming closer and closer together in 7 steps. Each step is arbitraly designated a number (15 broadest gap to 21 smallest gap) and the clarity of the formulation was given the value corresponding to the set of lines which were just visually resolvable through the sample.

All the formulations had a clarity at all storage temperatures of 19 or above; formulation F was the clearest (clarity 20), closely followed by formulation E.

**EXAMPLE 3**

| **INGREDIENT** | **% WT** |
|---|---|
| Sident 10 (abrasive silica) | 10.00 |
| Sorbitol (70%) | 57.31 |
| Sorbosil TL15 (thickening silica) | 8.00 |
| Polyethylene glycol MW 1500 | 5.00 |
| Sodium monofluorophosphate | 0.80 |
| Sodium lauryl sulphate | 1.70 |
| Sodium carboxymethylcellulose | 0.60 |
| Saccharin | 0.20 |
| Zinc Sulphate | 0.686 |
| Glycine | 0.3584 |
| Colouring agent | 0.012 |
| Alcohol | 1.00 |
| Triclosan | 0.30 |
| Flavour | 1.10 |
| Water | 12.934 |

The stability data for fluoride and zinc of this formulation were as follows:

| Water available | 28°C | 37°C |
|---|---|---|
| Fluorine (ppmF) | | |
| Initial | 1042 | 1042 |
| Month 2 | 1056 | 1033 |
| Month 3 | 1082 | 1087 |
| Month 6 | 1050 | 995 |

| Water available | 28°C | 37°C |
|---|---|---|
| Zinc (%) | | |
| Initial | 0.684 | 0.684 |
| Month 2 | 0.686 | 0.686 |
| Month 3 | 0.680 | 0.598 |
| Month 6 | 0.615 | 0.500 |

## Claims

1. A visually clear, gel-type dentifrice comprising in a liquid medium a zinc salt which is more water-soluble than zinc fluoride, an amino acid which can bind zinc, and a low refractive index type silica as abrasive agent and wherein the refractive index of the liquid medium closely matches the refractive index of the silica abrasive.

2. A dentifrice according to claim 1, wherein the zinc salt is zinc sulphate and the amino acid is glycine.

3. A dentifrice according to claim 1 or 2, wherein the silica has a refractive index of between 1.41 and 1.47.

4. A dentifrice according to claim 3, wherein the silica has a refractive index of between 1.435 and 1.445.

5. A dentifrice according to claims 1 - 4, wherein the dentifrice further comprises sodium monofluorophosphate.

6. A dentifrice according to claims 1 - 5, further comprising coloured particles suspended in the clear gel.

7. A dentifrice according to claims 1 - 6, further comprising coloured stripes.

## Patentansprüche

1. Visuell durchsichtige Zahnpasta vom Geltyp, umfassend in einem flüssigen Medium ein Zinksalz, welches in Wasser löslicher ist als Zinkfluorid, eine Aminosäure, die Zink binden kann, und ein Siliziumdioxid vom niedrigen Brechungsindex-Typ als Putzkörper, und worin der Brechungsindex des flüssigen Mediums genau mit dem Brechungsindex des Siliziumdioxid-Putzkörpers übereinstimmt.

2. Zahnpasta nach Anspruch 1, wobei das Zinksalz Zinksulfat ist und die Aminosäure Glycin ist.

3. Zahnpasta nach Anspruch 1 oder 2, wobei das Siliziumdioxid einen Brechungsindex zwischen 1,41 und 1,47 aufweist.

4. Zahnpasta nach Anspruch 3, wobei das Siliziumdioxid einen Brechungsindex zwischen 1,435 und 1,445 aufweist.

5. Zahnpasta nach Ansprüchen 1 - 4, wobei die Zahnpasta weiterhin Natriummonofluorophosphat umfasst.

6. Zahnpasta nach Ansprüchen 1 - 5, die weiterhin in dem durchsichtigen Gel suspendierte gefärbte Teilchen umfasst.

7. Zahnpasta nach Ansprüchen 1 - 6, die weiterhin gefärbte Streifen umfasst.

## Revendications

1. Dentifrice sous forme de gel optiquement clair comprenant dans un milieu liquide, un sel de zinc qui est plus soluble dans l'eau que le fluorure de zinc, un acide aminé qui peut lier le zinc, et une silice ayant un faible indice de réfraction en tant qu'agent abrasif, dans lequel l'indice de réfraction du milieu liquide est très proche de l'indice de réfraction de la silice abrasive.

2. Dentifrice selon la revendication 1, dans lequel le sel de zinc est du sulfate de zinc et l'acide aminé est de la glycine.

3. Dentifrice selon la revendication 1 ou 2, dans lequel la silice a un indice de réfraction compris entre 1,41 et 1,47.

4. Dentifrice selon la revendication 3, dans lequel la silice a un indice de réfraction compris entre 1,435 et 1,445.

5. Dentifrice selon les revendications 1 - 4, dans lequel le dentifrice comprend en outre du monofluorophosphate de sodium.

6. Dentifrice selon les revendications 1 - 5, comprenant en outre des particules colorées en suspension dans le gel clair.

7. Dentifrice selon les revendications 1 - 6, comprenant en outre des bandes colorées.
